# EUROPEAN PATENT SPECIFICATION

(11) **EP 4 206 220 B1**
(45) Date of publication and mention of the grant of the patent: **02.10.2024**
(21) Application number: 20953153.2
(22) Date of filing: 24.12.2020
(51) Int. Cl.: C07K 14/62, C12N 15/11, A61K 38/00, C12N 15/70, C12P 21/06

(54) **NOVEL PROINSULIN GLARGINE AND METHOD FOR PREPARING INSULIN GLARGINE THEREFROM**
PROINSULINGLARGIN UND VERFAHREN ZUR HERSTELLUNG VON INSULINGLARGIN DARAUS
NOUVELLE PRO-INSULINE GLARGINE ET PROCÉDÉ DE PRÉPARATION D'INSULINE GLARGINE À PARTIR DE CELLE-CI

(30) Priority: 11.09.2020 CN 202010952921
(43) Date of publication of application: 05.07.2023
(73) Proprietor: AMPHASTAR NANJING PHARMACEUTICALS, INC., Jiangsu 210038 (CN)
(72) Inventor: TANG, Chuangen, Nanjing, Jiangsu 210046 (CN); WANG, Jing, Nanjing, Jiangsu 210046 (CN); PAN, Shangshu, Nanjing, Jiangsu 210046 (CN); FAN, Xiaoyang, Nanjing, Jiangsu 210046 (CN); LIU, Xiaorui, Nanjing, Jiangsu 210046 (CN); CHEN, Song, Nanjing, Jiangsu 210046 (CN); ZHANG, Haoning, Nanjing, Jiangsu 210046 (CN)
(74) Representative: Ipside
(86) International application number: PCT/CN2020/138950
(87) International publication number: WO 2022/052368

(56) References cited:
- EP-A1- 3 845 240
- WO-A1-2012/115638
- WO-A1-2017/126984
- WO-A1-2020/051812
- CN-A- 102 015 762
- CN-A- 103 981 242
- US-A- 5 523 215
- DATABASE Geneseq [online] 7 September 2017 (2017-09-07), "Human insulin protein, SEQ ID 15.", retrieved from EBI accession no. GSP:BEE29061 Database accession no. BEE29061

## Description

### Technical Field

The present invention relates to novel proinsulin glargine and a method for preparing insulin glargine therefrom, and belongs to the technical field of recombinant protein preparation.

### Background of the Invention

Insulin is a hormone for regulating glucose metabolism in an animal body. This hormone is composed of 2 peptide chains, namely a chain A and a chain B; the chain A contains 21 amino acids, and the chain B contains 30 amino acids, a total of 51 amino acids. 4 cysteines, namely A₇ (Cys)-B₇ (Cys) and A₂₀ (Cys)-B₁₉ (Cys), form 2 disulfide bonds which are used for connecting the chain A and the chain B. An in-chain disulfide bond formed by A₆ (Cys) and A₁₁ (Cys) exists in the chain A. Diabetes mellitus is characterized in that the blood glucose level is increased due to insulin deficiency and/or increase of the yield of hepatic glucose, and the insulin is the only hormone which can reduce blood glucose in the body.

The overall goal of insulin analogue development is to simulate physiological insulin secretion to improve the blood glucose control of patients undergoing type 1 and type 2 diabetes (Berger M. A comment. Diabetes Res Clin Pract. 6: S25-S31, 1989; Sanlioglu AD et al., Clinical utility of insulin and insulin analogs. Islets 5(2): 67-78, 2013). Recent insulin analogues (natural insulin analogues) are prepared by adding amino acid residues or replacing amino acid residues on natural insulin molecules by genetic engineering or biochemical reaction, or modifying other functional groups. These modifications change the speed of biological drug efficiency by changing the pharmacological, pharmacokinetic and pharmacodynamic characteristics of insulin molecules, such as insulin aspart, insulin glargine and insulin lispro. Insulin glargine (US patent US5656722) is an insulin analogue with a long-acting effect. Glycine is used for replacing aspartic acid at A₂₁, and two arginine residues are added at the C terminal of the Chain B, so that the insulin glargine forms a precipitate (hexamer-microcrystal) during injection. The isoelectric point of insulin glargine is increased from pH 5.4 to pH 6.7, so that the molecules are water-soluble at acidic pH, and finally the hexamer of the insulin glargine is slowly dissociated into monomers. In a subcutaneous region with neutral pH, the formation of the hexamer causes the insulin to be slowly dissolved and absorbed from an injection site without a peak value, and provides a lasting action time of 24-26 h. The prolonging effect of the insulin glargine reduces the peak effect and reduces the risk of hypoglycemia. Compared with NPH neutral protamine zinc insulin, the insulin glargine shows a lower severe hypoglycemia occurrence rate (Sanlioglu AD et al., Clinical utility of insulin and insulin analogs. Islets 5(2): 67-78, 2013).

Human insulin is the first protein drug generated by a recombinant DNA technology. In 1978, human insulin was successfully expressed in the laboratory for the first time; and in 1982, the recombinant human insulin was approved as a therapeutic drug. The precursor protein of the recombinant human insulin is synthesized by genetically modified organisms, and is hydrolyzed and cleaved by protease to generate active insulin. Almost all insulin analogues on the market are modified from insulin human genes by a genetic engineering technology, and are generated in *Escherichia coli* or yeast.

One of the methods using transgenic *E. coli* is to respectively express the chain A and the chain B of insulin in *E. coli,* and then mix the sulfonated chain A and chain B in vitro to form an inter-chain disulfide bond (Rich, D.H. et al., Pierce Chemical Company, Rockford. Pp. 721-728, 1981. and Frank, B.H. et al., Pierce Chemical Company, Rockford. Pp. 729-738, 1981). However, this method has the defects that two separate fermentation processes are needed and a proper disulfide bond is formed. The improper disulfide bond formed between the sulfonated chain A and chain B may cause low yield of insulin.

A patent CN103981242A relates to a method for producing insulin by using copper/zinc superoxide dismutase (SOD) as a fusion peptide. The fusion peptide is composed of 64 amino acids, the first amino acid is Met, and the last amino acid is Arg; and meanwhile, all cysteine residues in the fusion peptide chain are substituted by serine residues. The amino acid sequence of the SOD fusion peptide fragment in the patent is disclosed as: MATKAVSVLKGDGPVQGIINFEQKESNGPVKVWGSIKGLTEGLHGFHVHEFGDNTAGSTSAGP R (SEQ ID NO: 1). The SOD fragment has 5 Lys residues which are natural cleavage sites of trypsin. In the subsequent enzyme digestion process, miscleavage impurities are easily generated, thereby resulting in low yield and purity.

A use method of proinsulin without a conserved binary amino acid terminal sequence in a C-peptide region has also been reported. For example, a proinsulin peptide structure involved in a US patent US6777207 is composed of shortened C-peptide (the length does not exceed 15 amino acid residues), and the C-peptide has two terminal amino acids used as glycine-arginine or glycine-lysine and is connected to a chain at the terminal of an A-carboxyl group. Opposite to the Chain B having 30 amino acids in the total length of natural human insulin, the proinsulin construct contains a Chain B having 29 amino acids. The potential influences of the shortened Chain B and the shortened C-peptide in human insulin production are not clear yet, but the amino acid at the B30 site needs to be connected through a transpeptidation reaction with low yield. A host cell mentioned in the patent is yeast, but no evidence indicates that the construct can be used for *E. coli.* Because the fermentation period of a yeast expression system is long, the expression efficiency is reduced, and the cost is increased. At present, it is urgently needed to design a more efficient and perfect structural sequence of novel proinsulin glargine and a process for preparing insulin glargine by using the structural sequence to solve the above problems in the prior art.

### Summary of the Invention

The present invention discloses novel proinsulin glargine capable of effectively improving a recombinant insulin glargine preparation process and a method for preparing insulin glargine by using same. The structure of the proinsulin glargine designed in the present invention includes an N-terminal fusion peptide sequence (SOD subjected to site-directed mutagenesis), an insulin Chain A modified by A₂₁ and a full-length human insulin Chain B containing two arginine residues. Compared with natural human insulin, the amino acid on the A₂₁ site in the insulin glargine Chain A is formed by substituting glycine for asparagine, and two arginine residues are added to a carboxyl terminal of the Chain B. The structure of the proinsulin glargine adopts a "0 C peptide" strategy, i.e. no C peptide sequence exists between the Chain B and the Chain A. The proinsulin glargine is expressed in *E. coli,* and subjected to renaturation with the fusion peptide under proper renaturation conditions; and then the fusion peptide is separated from insulin glargine molecule by trypsinase digestion, thereby obtaining the insulin glargine.

The proinsulin glargine designed in the present invention can be effectively folded into a natural structure in the presence of a SOD fragment fusion peptide subjected to site-directed mutagenesis, thereby improving the fermentation yield of the insulin glargine. When there is a proper protease digestion site, for the proinsulin glargine, the increase of a C peptide sequence possibly causes C peptide residues after enzyme digestion and influences the purification and yield. In the present invention, the novel proinsulin glargine adopts the "0 C peptide" strategy to effectively avoid the C peptide residues after enzyme digestion and minimize the mass loss in the digestion transformation step.

A first objective of the present invention is to provide novel proinsulin glargine, the amino acid sequence of which has the following structure:

R-R₁-(B₁-B₃₂)-(A₁-A₂₀)-A₂₁

wherein,
R-R₁ is a fusion peptide sequence, and the amino acid sequence of R is 
MATX1AVSVLKGDGPVQGIINFEQX2ESNGPVKVWGSIX3GLTEGLHGFHVHEFGDNTAGSTSA
GP;
X₁ is proline Pro(P) or histidine His(H); X₂ is proline Pro(P) or histidine His(H); X₃ is proline Pro(P) or histidine His(H);
R₁ is arginine Arg(R) or lysine Lys(K);
B₁-B₃₂ is formed by adding two arginine Arg residues (R) behind the C-terminal of the B₃₀ site of B₁-B₃₀ in the Chain B of natural human insulin;
A₁-A₂₀ is an insulin Chain A having 20 amino acids; and
A₂₁ is glycine (G).

In one implementation, the amino acid sequence of R is disclosed as MATPAVSVLKGDGPVQGIINFEQPESNGPVKVWGSIPGLTEGLHGFHVHEFGDNTAGSTSAGP (SEQ ID NO: 2); or

MATHAVSVLKGDGPVQGIINFEQHESNGPVKVWGSIHGLTEGLHGFHVHEFGDNTAGSTSAG
P (SEQ ID NO: 3).

In one implementation, the C-terminal of the fusion peptide sequence is connected with B₁-B3₂ through lysine residues or arginine residues.

In one implementation, the amino acid sequence of A₁-A₂₀ is disclosed as GIVEQCCTSICSLYQLENYC (SEQ ID NO: 4).

In one implementation, the amino acid sequence of B₁-B₃₀ is disclosed as FVNQHLCGSHLVEALYLVCGERGFFYTPKT (SEQ ID NO: 5).

In one implementation, two arginine residues are used for prolonging the Chain B, so that the amino acid sequence of B₁-B₃₂ is disclosed as FVNQHLCGSHLVEALYLVCGERGFFYTPKTRR.

In one implementation, the amino acid sequence of (B₁-B₃₂)-(A₁-A₂₀)-A₂₁ in the proinsulin glargine is disclosed as:
FVNQHLCGSHLVEALYLVCGERGFFYTPKTRRGIVEQCCTSICSLYQLENYCG (SEQ ID NO: 6).

In one implementation, the amino acid sequence of the proinsulin glargine R-R₁-(B₁-B₃₂)-(A₁-A₂₀)-A₂₁ is disclosed as any one of SEQ ID NO: 7, SEQ ID NO: 8, SEQ ID NO: 9 and SEQ ID NO: 10. 
MATPAVSVLKGDGPVQGIINFEQPESNGPVKVWGSIPGLTEGLHGFHVHEFGDNTAGSTS
AGPKFVNQHLCGSHLVEALYLVCGERGFFYTPKTRRGIVEQCCTSICSLYQLENYCG (SEQ ID
NO: 7);
 
MATPAVSVLKGDGPVQGIINFEQPESNGPVKVWGSIPGLTEGLHGFHVHEFGDNTAGSTS
AGPRFVNQHLCGSHLVEALYLVCGERGFFYTPKTRRGIVEQCCTSICSLYQLENYCG (SEQ ID
NO: 8);
 
MATHAVSVLKGDGPVQGIINFEQHESNGPVKVWGSIHGLTEGLHGFHVHEFGDNTAGSTS
AGPKFVNQHLCGSHLVEALYLVCGERGFFYTPKTRRGIVEQCCTSICSLYQLENYCG (SEQ ID
NO: 9);
 and

MATHAVSVLKGDGPVQGIINFEQHESNGPVKVWGSIHGLTEGLHGFHVHEFGDNTAGSTS
AGPRFVNQHLCGSHLVEALYLVCGERGFFYTPKTRRGIVEQCCTSICSLYQLENYCG (SEQ ID
NO: 10).

A second objective of the present invention is to provide a DNA for encoding proinsulin glargine.

In one implementation, the DNA has a nucleotide sequence disclosed as any one of SEQ ID NO: 11-14.

In one implementation, the SEQ ID NO: 11, SEQ ID NO: 12, SEQ ID NO: 13 and SEQ ID NO: 14 respectively encode amino acid sequences disclosed as SEQ ID NO: 7, SEQ ID NO: 8, SEQ ID NO: 9 and SEQ ID NO: 10.

A third objective of the present invention is to provide an expression vector containing the DNA.

In one implementation, the expression vector includes but is not limited to pET series plasmids.

A fourth objective of the present invention is to provide non-plant cells for expressing the proinsulin glargine, including but not limited to eukaryotic cells or prokaryotic cells.

In one implementation, the cells express the DNA for encoding the proinsulin glargine, or the expression vector is introduced.

In one implementation, the microbial cells include but are not limited to *E. coli*, *Bacillus subtilis* and *Saccharomyces cerevisiae cells.*

In one implementation, the cells include mammalian cells or insect cells.

In one implementation, the cells are prokaryotic cells, including but not limited to *E. coli*, *B. subtilis* or any improved variety more suitable for recombinant protein expression, such as *E*. *coli* DH5a, K12JM107, W3110, BL21 (DE3), Rosetta or other strains.

In one implementation, the cells are recombinant *E. coli,* and contain pET28a plasmids carrying proinsulin glargine encoding genes.

A fifth objective of the present invention is to provide a method for producing insulin glargine by fermenting the recombinant *E. coli.*

In one implementation, the method includes the following steps: inoculating the recombinant *E. coli* into a BFM culture medium, and fermenting at 35-37°C for at least 20 h.

In one implementation, the BFM culture medium contains diammonium hydrogen phosphate, ammonium chloride, potassium dihydrogen phosphate, magnesium sulfate heptahydrate, citric acid monohydrate, glucose, yeast powder and microelements.

In one implementation, the inoculation is to inoculate a recombinant *E. coli* seed solution.

In one implementation, the seed solution is subjected to two-stage fermentation: the first-stage fermentation is carried out in an LB culture medium at 35-37°C for 6-10 h to obtain a primary seed solution; and then the primary seed solution is transferred into the BFM culture medium according to the inoculation size of 0.2%, and cultured for 6-10 h to obtain a secondary seed solution.

In one implementation, the method also includes the following steps: carrying out enzyme digestion, renaturation and purification on the fermented insulin glargine.

In one implementation, the method includes the following steps:
(1) culturing and fermenting the recombinant *E*. *coli* to express proinsulin glargine;
(2) releasing and dissolving an inclusion body and renaturing the proinsulin glargine;
(3) modifying the refolded proinsulin glargine by using citraconic anhydride;
(4) digesting the proinsulin glargine modified in step (3) by using trypsin, and carrying out acidic hydrolysis to obtain insulin glargine;
(5) purifying the insulin glargine; and
(6) precipitating, washing, dissolving, filtering and freeze-drying the insulin glargine.

In one implementation, in step (2), lysozyme treatment and high-pressure homogenization are carried out.

In one implementation, in step (2), diluting and renaturing are carried out at 15-25°C under the pH of 10.0-11.6.

In one implementation, in step (3), citraconic anhydride is added into the renatured proinsulin glargine.

In one implementation, in step (4), trypsin digestion transformation is carried out on the modified proinsulin glargine containing the sequence according to claim 1, and the fusion peptide is removed to obtain the insulin glargine of which lysine at a B₂₉ site is modified by the citraconic anhydride residues.

In one implementation, in step (4), acidic hydrolysis is carried out under the pH of 1.5-2.5 to remove the citraconic anhydride residues, thereby obtaining the insulin glargine.

In one implementation, in step (5), the insulin glargine is purified by ion exchange chromatography to obtain high-purity insulin glargine.

In one implementation, in step (5), the insulin glargine is further purified by preparative HPLC to obtain higher-purity insulin glargine.

In one implementation, the high-purity insulin glargine is crystallized and dried to form a final insulin glargine active pharmaceutical ingredient.

In one implementation, the above optimized gene is connected to a proper vector, such as pTAC expression plasmid series, pGEX series or pET series, preferably pET series plasmid, and more preferably pET-28a plasmid; and the plasmid can transfect K12 JM109 engineering bacteria or K12 W110 engineering bacteria to form expression clone. In another preferable implementation, the expression plasmid is transfected into BL21 (DE3) engineering bacteria.

In one implementation, the recombinant *E. coli* is cultured to a proper concentration through a shake flask or fermentation tank, and then is used for inducing the expression of the proinsulin glargine.

In one implementation, the cells containing the inclusion body of the proinsulin glargine are treated by lysozyme treatment and high-pressure homogenization for cracking; and the separated inclusion body is washed by a solution containing a detergent or low-concentration chaotropic agent and then is dissolved by a high-pH buffer solution.

In one implementation, the pH value of the dissolution buffer solution is 11.6-12.4, and the dissolution buffer solution contains Tris, EDTA and L-cysteine; and the concentration of the Tris is 10-50 mM, the concentration of the EDTA is 0.05-1.0 mM, and the concentration of the L-cysteine is 0.25-5.0 mM.

In one implementation, the concentration of the Tris is 20-30 mM; the concentration of the EDTA is 0.05-0.25 mM; the concentration of the L-cysteine is 0.25-1.0 mM; and the pH is 11.8-12.2.

In one implementation, the temperature of the dissolution buffer solution is 10-30°C or 15-25°C; and the dissolution time of the inclusion body is 10-120 min or 10-60 min.

In one implementation, the pH value of the dissolution buffer solution is 10.0-11.6 or 10.8-11.4; the temperature of the solution is about 10-25°C or 15-20°C; the concentration of the total protein is 1-10 g/L or 1-7 g/L; and the renaturation duration is 12-48 h or 24-36 h.

In one implementation, a protective reagent is used for modifying the proinsulin glargine before enzyme digestion; and the protective reagent can be an electrophilic reagent which can easily react with the γ-NH₂ group of B29-Lys, such as acid anhydride, including but not limited to acetic anhydride, citric anhydride or citraconic anhydride.

In one implementation, excessive molar ratio of citraconic anhydride is added into the proinsulin glargine; optionally, 10 times or more molar amounts of citraconic anhydride are added into the proinsulin glargine; or 20 times or more molar amounts of citraconic anhydride are added into the proinsulin glargine.

In one implementation, the reaction temperature for adding the protective reagent for modification is 15-25°C, the pH value is 8.0-9.0, and the duration is 2-8 h.

In one implementation, ethanolamine can be added to neutralize excessive citraconic anhydride after the modification is finished, the volume of the ethanolamine is 40-80% of the citraconic anhydride, and the termination time is 10-30 min.

In one implementation, the protein concentration of the renaturation solution is regulated to 1-7 g/L. 0.2 vol% of citraconic anhydride is added into the renaturation solution, the pH is regulated to 8.5, and then reaction is carried out at 20°C for 4 h; and after modification is finished, ethanolamine which accounts for 60 vol% of the citraconic anhydride for modification is added for neutralizing, the pH is regulated to 9.0, and then neutralizing is carried out for 30 min. Trypsin (preferably bovine trypsin) with the final concentration of 220 U/g protein is added into citraconic acid modified proinsulin glargine, the pH is regulated to 9.0, and digesting is carried out at 20°C for 24 h to obtain insulin glargine with the citraconic acid residues. The process is monitored through HPLC-RP (C18). Once the enzyme digestion reaction finishes, hydrochloric acid is added, the pH is regulated to 2.0-2.5, and then the enzyme digestion reaction can be terminated. The solution is kept at the low pH value for 24 h to hydrolyze the citraconic acid residues on B29-Lys so as to obtain the insulin glargine.

In one implementation, after the protein concentration of the renaturation solution is regulated to 4 g/L, 0.05 vol% of citraconic anhydride is directly added into the renaturation protein solution, the pH is regulated to 8.5, and then reaction is carried out at 20°C for 2 h. After modification is finished, ethanolamine which accounts for 60 vol% of the citraconic anhydride for modification is added for neutralizing, the pH is regulated to 9.4, and then neutralizing is carried out for 15 min. Trypsin with the final concentration of 0.063 mg/g protein is added into citraconic acid modified insulin glargine, the pH is regulated to 9.0, and then reaction is carried out at 20°C for 24 h to obtain the insulin glargine with the citraconic acid residues. The process is monitored through HPLC-RP (C18). Once the enzyme digestion reaction is finished, hydrochloric acid is added, and the pH is regulated to 2.0-2.5 to terminate the reaction. The solution is kept at the low pH value for 12 h, and hydrolyzing is carried out to remove the citraconic acid residues on B29-Lys so as to obtain the insulin glargine.

In one implementation, after trypsin enzymolysis, zinc ions with the final concentration of 1-10 mM are added, and the pH is regulated to 5.5-6.5, so that the insulin glargine forms a precipitate and is separated out; and the insulin glargine is purified by a proper method to obtain a final insulin glargine product.

In one implementation, the prepared RP-HPLC and ammonium dihydrogen phosphate buffer system is used for one-step purification, and the concentration of the ammonium dihydrogen phosphate is 0.05-0.3 M or 0.05-0.2 M; the pH value is 2.0-5.0; the organic modifier can be ethanol, methanol or acetonitrile; and in a linear concentration gradient of an organic solvent, the insulin glargine is eluted.

In one implementation, the prepared RP-HPLC and Tris-HCl buffer system is used for final purification, and the Tris concentration is 0.02-0.3 M or 0.02-0.2 M; the pH value is 7.0-9.0; the organic modifier can be ethanol, methanol or acetonitrile; and in the linear concentration gradient of the organic solvent, the insulin glargine is eluted.

In one implementation, the insulin glargine eluted from RP-HPLC is precipitated by an isoelectric precipitation method, and the precipitate is collected. Resuspension washing is carried out on the collected precipitate by a 0-1.5% sodium chloride solution; the washed and centrifuged wet solid is dissolved by 40-200 mM of a hydrochloric acid solution; the concentration of the insulin glargine is regulated to 20-40 mg/mL; the pH is regulated to 3.0-5.0; filtering is carried out; and then freeze drying is carried out on the filtrate to obtain the insulin glargine active pharmaceutical ingredient existing in a crystal or cured API form.

The present invention further claims protection on application of the method in preparation of insulin glargine or drugs containing insulin glargine.

### Beneficial effects:

According to the present invention, the SOD fragment subjected to site-directed mutagenesis is used as the fusion peptide, and the "0 C peptide" strategy is adopted, so high fermentation yield can be obtained, and the fermentation yield of the insulin glargine is increased by 75% or above, up to 78%;

According to the present invention, through the "0 C peptide" strategy, remaining of the C peptide residues are avoided, and quality loss in the step of enzyme transformation is reduced; in this patent application, impurities caused by wrong refolding and wrong enzyme digestion are reduced, so that the yield and purity of the final product are improved, the chromatographic purity of a main peak reaches 99.4 or above, and the maximum single impurity content is not greater than 0.16%; in a preferred embodiment, the chromatographic purity of the main peak reaches 99.9%, and the maximum single impurity content is controlled to be 0.05% or below; and the chromatographic purity of the main peak is increased by 7% compared with the comparative example, and the maximum single impurity content is decreased from 2.15% to not greater than 0.16% compared with the comparative example, which is obviously reduced by one order of magnitude.

According to the preparation method provided by the present invention, bovine trypsin is used for enzyme digestion, and compared with porcine trypsin, the enzyme digestion transformation rate can be increased by 16%. Therefore, the production cost for preparation of high-quality insulin glargine can be greatly reduced.

### Brief description of the Drawings

FIG. 1 is a purification liquid chromatogram of a strain constructed by using a gene sequence SEQ ID NO: 11 according to a specific example 6 of the present invention.
FIG. 2 is a purification liquid chromatogram of a strain constructed by using a gene sequence SEQ ID NO: 12 according to a specific example 7 of the present invention.
FIG. 3 is a purification liquid chromatogram of a strain constructed by using a gene sequence SEQ ID NO: 13 according to a specific example 8 of the present invention.
FIGS. 4A, 4B and 4C are liquid chromatograms obtained after renaturation of a strain WCB01 constructed by using a gene sequence SEQ ID NO: 10 according to specific examples 1-3 of the present invention.
FIGS. 5A, 5B and 5C are liquid chromatograms obtained after renaturation of a strain WCB02 constructed by using a gene sequence SEQ ID NO: 15 according to specific examples 1-3 of the present invention.
FIG. 6 is a liquid chromatogram obtained after renaturation of a strain WCB03 constructed by using a gene sequence SEQ ID NO: 16 according to specific examples 1-3 of the present invention.
FIG. 7 is a liquid chromatogram obtained after renaturation of a strain WCB04 constructed by using a gene sequence SEQ ID NO: 17 according to specific examples 1-3 of the present invention.
FIG. 8 is a liquid chromatogram obtained after renaturation of a strain WCB05 constructed by using a gene sequence SEQ ID NO: 18 according to specific examples 1-3 of the present invention.
FIG. 9A is a liquid chromatogram obtained after purification of a strain WCB01 constructed by using a gene sequence SEQ ID NO: 10 according to specific examples 1-5 of the present invention.
FIG. 9B is a liquid chromatogram obtained after purification of a strain WCB02 constructed by using a gene sequence SEQ ID NO: 15 according to specific comparative examples 1-3 of the present invention.
FIG. 10A is a liquid chromatogram of digestion effect of bovine trypsin on proinsulin glargine according to a specific example 4 of the present invention.
FIG. 10B is a liquid chromatogram of digestion effect of porcine trypsin on proinsulin glargine according to a specific comparative example 4 of the present invention.

### Detailed Description of Embodiments

The materials, reagents and the like used in the following embodiments can be obtained commercially without special instructions.

### Embodiment 1: Structure design of novel proinsulin glargine

A protein sequence of proinsulin glargine shown in a formula I was designed:

R-R₁-(B₁-B₃₂)-(A₁-A₂₀)-A₂₁

The improved sequence of the proinsulin glargine adopted a "0 C peptide" strategy, namely, no amino acid sequence existed between a chain B and a chain A. An N-terminal lead amino acid sequence can enhance expression, protect the proinsulin glargine and prevent the proinsulin glargine from being degraded by *E. coli.* The amino acid sequence of R in a fusion peptide R-R₁ is
MATHAVSVLKGDGPVQGIINFEQHESNGPVKVWGSIHGLTEGLHGFHVHEFGDNTAGSTSAG P (shown as SEQ ID NO: 3). A C terminal of the amino acid sequence of the fusion peptide was connected to the chain B of the insulin glargine through arginine or lysine residues, and finally, the fusion peptide was removed through trypsin cracking.

### Embodiment 2: Construction of a recombinant plasmid containing a novel proinsulin glargine encoding gene

A sequence of novel proinsulin glargine was designed according to the method in the Embodiment 1: the sequence of (B₁-B₃₂)-(A₁-A₂₀)-A₂₁ in the novel proinsulin glargine was SEQ ID NO: 6. The complete sequence of proinsulin glargine with fusion peptide was disclosed as SEQ ID NO: 10. In order to ensure that the fusion protein disclosed as SEQ ID NO: 10 could be effectively expressed in *E. coli,* a genetic codon was optimized. The optimized gene sequence is disclosed as SEQ ID NO: 14, and contains a 5' Ncol site (CCATGG) and 1 3' Hind III site (AAGCTT).

A DNA fragment shown as SEQ ID NO: 14 was chemically synthesized by commercial CRO Company, cracked by *Ncol* and *Hind III* restriction enzymes, inserted into a pET-28a expression vector cracked by the same restriction enzyme, and connected by a ligase to form a pET-PIG-1 expression vector.

### Embodiment 3: Construction of recombinant E. coli expressing novel proinsulin glargine

The recombinant expression vector pET-PIG-1 constructed in the Embodiment 2 was transfected into *E. coli* BL21 (DE3) competent cells. Positive clones were screened through Kanamycin resistance and confirmed by using DNA sequencing. The positive clones were cultured and amplified at 37°C, and a sterile culture medium and glycerol were added into the cells. 1 mL of cell culture solution was transferred into a sterile ampoule, and stored at -80°C to form a proinsulin glargine working seed bank (WCB01).

### Embodiment 4: Expression of proinsulin glargine fusion protein

The WCB01 obtained in the Embodiment 3 was inoculated into an MLB culture medium (containing 15 g/L of yeast powder and 5 g/L of sodium chloride) according to the inoculation size of 0.2%, and cultured under 37°C at 250 rpm for 6-14 h to obtain a primary seed solution. The primary seed solution was inoculated into a BFM culture medium which containing 6 g/L of diammonium hydrogen phosphate, 4 g/L of ammonium chloride, 13.5 g/L of potassium dihydrogen phosphate, 1.39 g/L of magnesium sulfate heptahydrate, 2.8 g/L of citric acid monohydrate, 8 g/L of glucose, 3 g/L of yeast powder and 1 mUL of microelement solution (containing 10 g/L of ferrous sulfate heptahydrate, 1.1 g/L of zinc chloride, 1.0 g/L of copper sulfate pentahydrate, 0.4 g/L of manganese chloride tetrahydrate, 0.2 g/L of boric acid, 2.7 g/L of calcium chloride and 0.2 g/L of sodium molybdate) according to the inoculation size of 0.2%, and further cultured for 8-16 h to obtain a secondary seed solution. Then the secondary seed solution was inoculated into the BFM culture medium in a fermentation tank according to the volume ratio of 1:10, and continuously cultured at a growth temperature of 30-39°C under conditions of the growth dissolved oxygen content of 10-50% and the growth pH of 6.0-7.3 for 12-18 h until the OD₆₀₀ of fermentation liquid was 100-200; and IPTG with the final concentration of 0.1-1.0 mM was added into the fermentation tank to induce the expression of the proinsulin glargine, and other growth conditions were not changed. Continuous inducing was carried out for 8-16 h, and thalli were collected by using a centrifuge.

Thalli containing inclusion body of the proinsulin glargine were resuspended by using a buffer solution with pH of 8.0 and containing 25 mM of Tris and 10 mM of EDTA, and the concentration of the thalli is controlled to 200 g/L. The thalli were subjected to lysozyme treatment and high-pressure homogenization for cracking, a thallus lysate was centrifuged, the inclusion body precipitate was collected, and a supernatant was removed.

The inclusion body was washed by using a washing solution with pH of 8.0 and containing 25 mM of Tris, 1 M of urea and 1% of Tween 20. After washing, the inclusion body was resuspended by using a buffer solution containing 25 mM of Tris, 0.1 mM of EDTA and 0.5 mM of L-cysteine, the pH was regulated to 12.0, and dissolving was carried out at a temperature of 15°C for 50 min. The dissolved solution is named as an inclusion body dissolving solution.

### Embodiment 5: Renaturation of proinsulin glargine

The WCB01 inclusion body dissolving solution prepared in the Embodiment 4 was filtered through a 1 µm PP filter element, the temperature was controlled at 20°C, the pH was regulated to 11.0, and then renaturation was carried out for 32 h to obtain renatured proinsulin glargine.

### Embodiment 6: Preparation of insulin glargine by enzyme digestion transformation and purification

### (1) Preparation of insulin glargine by enzyme digestion

After renaturation, 0.2 vol% of citraconic anhydride was added into the renaturation solution for modifying. The pH of the renaturation solution was regulated to 8.5, and the renaturation solution was stirred to modify for 2 h. After modification, ethanolamine which accounts for 60% of the citraconic anhydride for modification was added to neutralize excessive citraconic anhydride, the pH was regulated to 9.4, and neutralizing was carried out for 15 min. Bovine trypsin with the final concentration of 0.063 mg bovine trypsin/g protein was directly added, the pH was regulated to 9.0, and enzyme digestion was carried out at 20°C for 24 h. Fusion peptide was removed to obtain citraconic anhydride modified insulin glargine. The enzyme digestion engineering was monitored by RP-HPLC (C18). After enzyme digestion, the pH was regulated to 2.0 with hydrochloric acid to terminate the enzyme digestion reaction. The pH was kept at 2.0 for 12 h, and citraconic anhydride modified lysine at a B₂₉ site was hydrolyzed to obtain the insulin glargine. After hydrolysis, zinc chloride with the final concentration of 3 mM was added, and the pH was regulated to 6.0, so that the insulin glargine forms a flocculent precipitate.

### (2) Purification of insulin glargine

The insulin glargine precipitate obtained after enzyme digestion and hydrolysis in step (1) was dissolved by 3 vol% acetic acid under pH of 3.5. The dissolved insulin glargine was loaded as a sample onto a cationic chromatographic column, and balanced with a buffer solution. The insulin glargine could be eluted by 30% isopropanol and 1.0 M of sodium chloride in a linear gradient manner. After cationic chromatography purification, the pH was regulated to 7.3 by zinc chloride with the final concentration of 3 mM so as to form flocculent precipitate of the insulin glargine. The above operations were repeated 2 times.

The insulin glargine purified by cationic chromatography was loaded onto a reversed-phase preparative chromatographic column. 0.1 M of ammonium dihydrogen phosphate and acetonitrile were mixed in a ratio of 9:1, and then the pH was regulated to 3.5 to obtain a solution-balanced chromatographic column. An elution buffer solution was a solution obtained by mixing a 0.1 M of diammonium hydrogen phosphate-10% acetonitrile mixed solution with the pH of 3.5 and 60% acetonitrile in different proportions. The insulin glargine was eluted by using the linear gradient of the elution buffer solution. The purity of the insulin glargine in the obtained insulin glargine eluate was 97%. After the first reversed-phase chromatographic purification, the pH was regulated to 7.3 by zinc chloride with the final concentration of 3 mM so that the insulin glargine formed the flocculent precipitate. The insulin glargine precipitate was dissolved with 3% acetic acid under the pH value of 3.5. The dissolved insulin glargine was loaded onto the reversed-phase preparative chromatographic column as a sample. 0.05 M of Tris and acetonitrile were mixed in a ratio of 9:1, and the pH was regulated to 8.5 to obtain a solution-balanced chromatographic column. The elution buffer solution was a solution obtained by mixing a 0.05 M of Tris-10% acetonitrile mixed solution with the pH of 8.5 and 60% acetonitrile in different proportions. The insulin glargine was eluted by using the linear gradient of the elution buffer solution, and the purity of the insulin glargine in the insulin glargine eluate was 99.9% by measurement. After the second reversed-phase chromatographic purification, the pH was regulated to 7.3 by 100 mM of a hydrochloric acid solution so that the insulin glargine formed the flocculent precipitate. The collected precipitate was subjected to resuspension washing by 0.3% of a sodium chloride solution (pH of 7.0) 3 times, and then centrifuged to obtain a wet insulin glargine solid.

### Embodiment 7 Preparation of insulin glargine active pharmaceutical ingredient

The wet insulin glargine solid prepared in the Embodiment 6 was dissolved by using 100 mM of a hydrochloric acid aqueous solution until the concentration was 30 mg/mL; the pH was regulated to 4.0; filtering was carried out by using a 0.22 µm PES filter membrane; the filtered insulin glargine solution was transferred into a freeze dryer, and the set parameters of the freeze drying procedure were as follows:
1) shelf refrigeration before feeding: the temperature was set to be -30°C;
2) refrigeration control: the temperature was set to be -30°C, the time was set to be 240 min, and the duration was set to be 240 min;
3) refrigeration of a water catcher: the temperature was set to be -50°C, and the duration was set to be 10 min;
4) pre-vacuumizing: pre-vacuumizing was carried out to reach 0.2000 mbar, the alarm vacuum was to be 0.5000 mbar, and the alarm vacuum duration was set to be 10 s;
5) primary drying: the temperature was set to be -10°C, the time was set to be 240 min, the duration was set to be 3,600 min, and the vacuum was set to be 0.1800 mbar; and
6) vacuum drying: the temperature was set to be 25°C, the time was set to be 480 min, the duration was set to be 240 min, and the vacuum was set to be 0.1800 mbar.

The purity of the final insulin glargine active pharmaceutical ingredient obtained by the treatment above was 99.9% or above.

### Embodiment 8 Preparation of insulin glargine

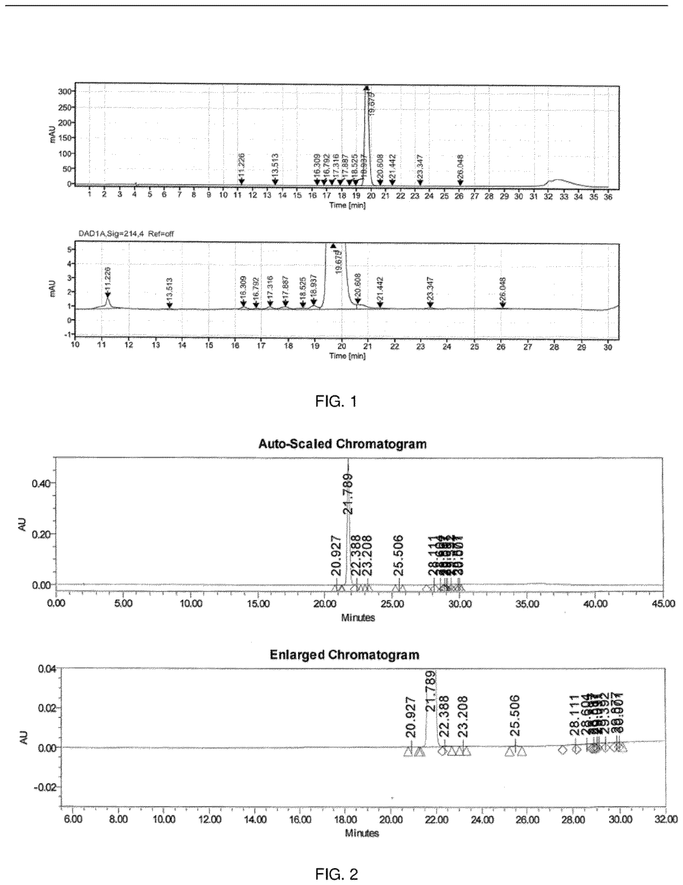

The specific implementation is the same as the Embodiments 2-6. The difference is as follows: the complete sequence of the proinsulin glargine with the fusion peptide is disclosed as SEQ ID NO: 7, and the gene sequence for encoding the proinsulin glargine with the fusion peptide is disclosed as SEQ ID NO: 11. The expression level of the fusion protein of the proinsulin glargine produced by fermenting the constructed recombinant *E*. *coli* under the same conditions is 5.8 g/L; and the HPLC detection on the purified product indicates that the high main peak chromatographic purity is 99.42% and the maximum single impurity chromatographic purity is 0.16% (see FIG. 1 and Table 1 for details).

**Table 1 Main peak areas and retention time in chromatography detection**

| # | Retention time | Peak area | % peak area |
|---|---|---|---|
| 1 | 11.226 | 12.93 | 0.16 |
| 2 | 13.513 | 0.79 | 0.01 |
| 3 | 16.309 | 3.22 | 0.04 |
| 4 | 16.792 | 1.10 | 0.01 |
| 5 | 17.316 | 3.26 | 0.04 |
| 6 | 17.887 | 4.05 | 0.05 |
| 7 | 18.525 | 2.18 | 0.03 |
| 8 | 18.937 | 4.83 | 0.06 |
| 9 | 19.675 | 7828.73 | 99.42 |

### Embodiment 9 Preparation of insulin glargine

The specific implementation is the same as the Embodiments 2-6. The difference is as follows: the complete sequence of the proinsulin glargine with the fusion peptide is disclosed as SEQ ID NO: 8, and the gene sequence for encoding the proinsulin glargine with the fusion peptide is disclosed as SEQ ID NO: 12. The expression level of the fusion protein of the proinsulin glargine produced by fermenting the constructed recombinant *E. coli* under the same conditions is 6.1 g/L; and the HPLC detection on the purified product indicates that the high main peak chromatographic purity is 99.59% and the maximum single impurity chromatographic purity is 0.14% (see FIG. 2 and Table 2 for details).

**Table 2 Main peak areas and retention time in chromatography detection**

| # | Retention time | Peak area | % peak area |
|---|---|---|---|
| 1 | 20.927 | 1134 | 0.02 |
| 2 | 21.789 | 6077592 | 99.59 |
| 3 | 22.388 | 4444 | 0.07 |
| 4 | 23.208 | 1540 | 0.03 |
| 5 | 25.206 | 8487 | 0.14 |
| 6 | 28.111 | 1759 | 0.03 |
| 7 | 28.604 | 2850 | 0.05 |
| 8 | 28.897 | 321 | 0.01 |
| 9 | 29.017 | 318 | 0.01 |
| 10 | 29.091 | 1534 | 0.03 |
| 11 | 29.392 | 1147 | 0.02 |
| 12 | 29.877 | 1119 | 0.02 |
| 13 | 30.001 | 602 | 0.01 |

### Embodiment 10 Preparation of insulin glargine

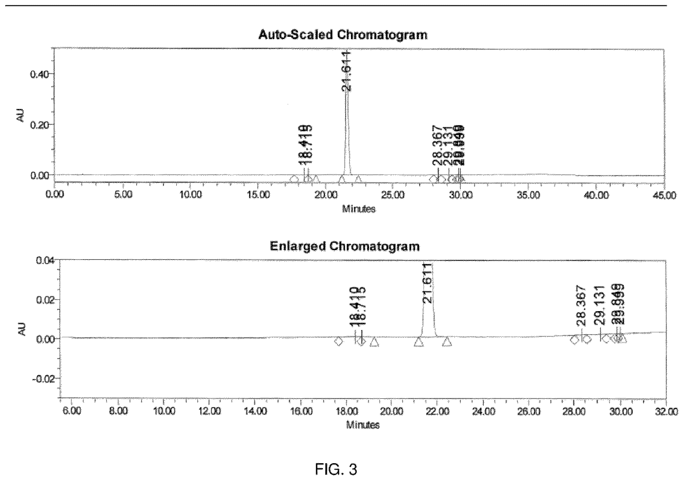

The specific implementation is the same as the Embodiments 2-6. The difference is as follows: the complete sequence of the proinsulin glargine with the fusion peptide is disclosed as SEQ ID NO: 9, and the gene sequence for encoding the proinsulin glargine with the fusion peptide is disclosed as SEQ ID NO: 13. The expression level of the fusion protein of the proinsulin glargine produced by fermenting the constructed recombinant *E*. *coli* under the same conditions is 6.0 g/L; and the HPLC detection on the purified product indicates that the high main peak chromatographic purity is 99.80% and the maximum single impurity chromatographic purity is 0.09% (see FIG. 3 and Table 3 for details).

**Table 3 Main peak areas and retention time in chromatography detection**

| # | Retention time | Peak area | % peak area |
|---|---|---|---|
| 1 | 18.410 | 6105 | 0.09 |
| 2 | 17.715 | 1916 | 0.03 |
| 3 | 21.611 | 6832023 | 99.80 |
| 4 | 28.367 | 1690 | 0.02 |
| 5 | 29.131 | 2405 | 0.04 |
| 6 | 29.849 | 813 | 0.01 |
| 7 | 29.999 | 737 | 0.01 |

### Comparative example 1: Construction of non-optimized recombinant E. coli of proinsulin glargine

The specific implementation is the same as the Embodiments 2-3. The difference is that the amino acid sequence in the fusion peptide R-R₁ is replaced by MATKAVSVLKGDGPVQGIINFEQKESNGPVKVWGSIKGLTEGLHGFHVHEFGDNTAGSTSAGP R (SEQ ID NO: 1), and a DNA segment of the whole amino acid sequence for encoding the SEQ ID NO: 1 is correspondingly adjusted to:

5'-
ATGGCGACGAAAGCCGTGAGCGTGCTGAAGGGCGACGGCCCAGTGCAGGGCATCATCAA
TTTCGAGCAGAAAGAAAGTAATGGACCAGTGAAGGTGTGGGGAAGCATTAAAGGACTGAC
TGAAGGCCTGCATGGATTCCATGTTCATGAGTTTGGAGATAATACAGCTGGCTCTACCAGT
GCAGGTCCGAAATTTGTGAACCAGCATCTGTGCGGCAGCCATCTGGTGGAAGCGCTGTAT
CTGGTGTGCGGCGAACGCGGCTTCTTTTATACCCCGAAAACCCGCCGCGGCATTGTGGAA
CAGTGCTGCACCAGCATTTGCAGCCTGTATCAGCTGGAAAATTATTGCGGCTAA-3' (SEQ ID
NO: 22).

The complete sequence of the proinsulin glargine with the fusion protein is disclosed as

MATKAVSVLKGDGPVQGIINFEQKESNGPVKVWGSIKGLTEGLHGFHVHEFGDNTAGSTSAGP
KFVNQHLCGSHLVEALYLVCGERGFFYTPKTRRGIVEQCCTSICSLYQLENYCG (SEQ ID NO:
15).

Then enzyme digestion treatment was carried out on the nucleotide sequence for encoding the above amino acid sequence and plasmid pET-28a by using Ncol and Hind III restriction enzymes, and the segment subjected to enzyme digestion was connected with a vector to obtain a recombinant expression vector pET-PIG-2.

The recombinant expression vector pET-PIG-2 was transformed into *E. coli* BL21 (DE3) competent cells. Positive clones were screened through Kanamycin resistance and confirmed by using DNA sequencing. The positive clones were cultured and amplified, and a sterile culture medium and glycerol were added into the cells. 1 mL of cell culture solution was transferred into a sterile ampoule, and stored at -80°C to form a proinsulin glargine working seed bank (WCB02).

### Comparative example 2: Expression of proinsulin glargine fusion protein

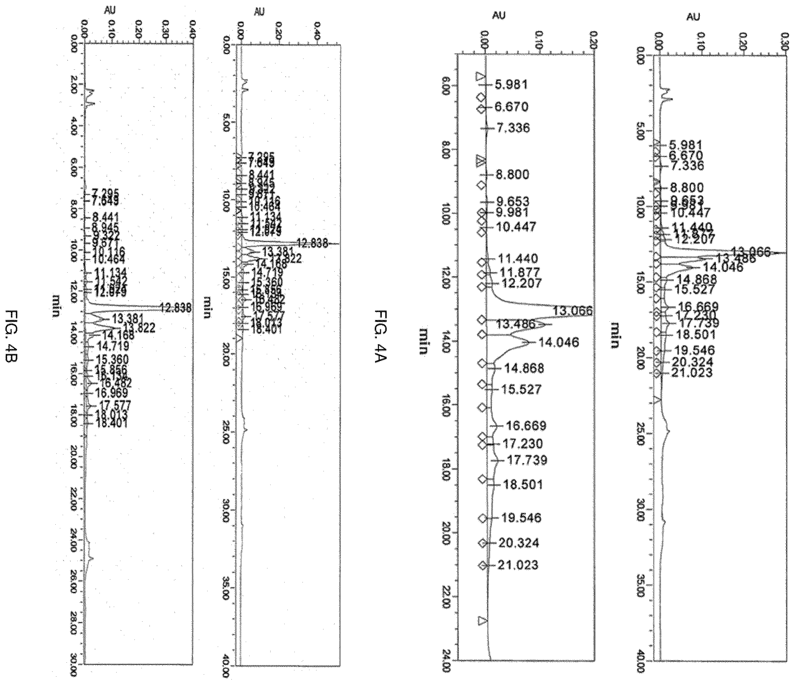

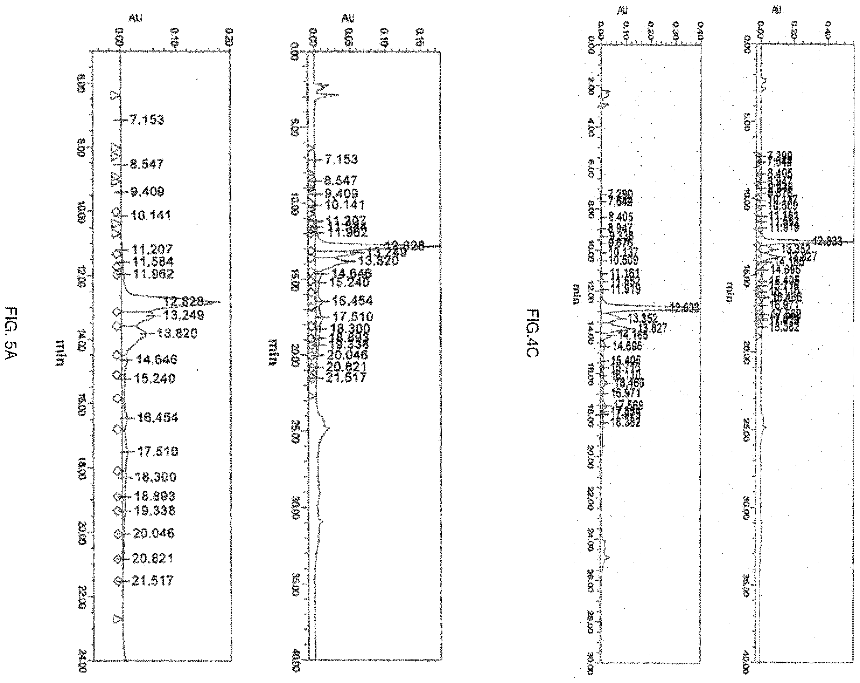

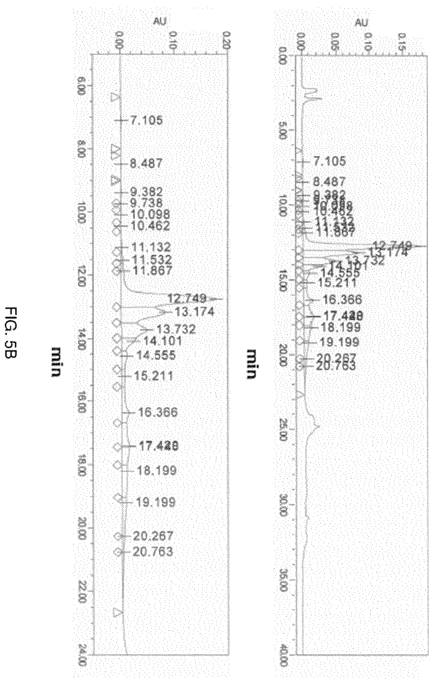

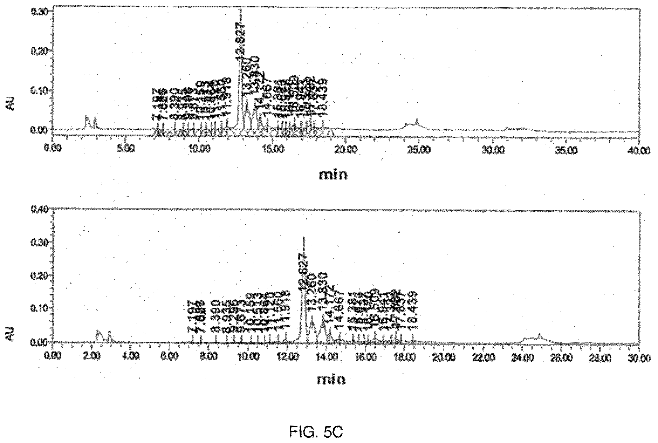

The recombinant bacteria WCB02 obtained in the Comparative example 1 were cultured in three batches according to the method in the Embodiment 4, and processed to obtain an inclusion body dissolving solution; and then, renaturation was carried out according to the method in the Embodiment 5. The yields of proinsulin glargine renaturation precursors obtained by fermenting WCB01 and WCB02 were respectively detected by using HPLC. The results of three groups of parallel experiments are shown in Table 4. The WCB01 renaturation liquid chromatography is shown in the FIGS. 4A, 4B and 4C, and related data are shown in Tables 4A, 4B and 4C; and the WCB02 renaturation liquid chromatography is shown in FIGS. 5A, 5B and 5C, and the related data are shown in Tables 5A, 5B and 5C.

**Table 4 Yields of proinsulin glargine renaturation precursor after different insulin glargine sequence expressions**

| # | **Yield under use of WCB01 (g/L)** | **Yield under use of WCB02 (g/L)** | **Rate of increase of WCB01 relative to WCB02** |
|---|---|---|---|
| 1 | 6.2 | 3.5 | 77% |
| 2 | 6.3 | 3.6 | 75% |
| 3 | 6.6 | 3.7 | 78% |

The result in Table 1 shows that by using the SOD fragment subjected to site-directed mutagenesis as the fusion peptide and adopting the preferred sequence SEQ ID NO: 10 of the "0 C peptide" strategy, more effective expression and more stable high fermentation yield can be obtained, and specifically, the fermentation yield of insulin glargine is increased by 75% or above, and is maximally increased by 78%.

**Table 4A Main peak areas and retention time in chromatography detection**

| # | Retention time | Peak area | % peak area |
|---|---|---|---|
| 1 | 5.981 | 3728 | 0.02 |
| 2 | 6.670 | 2863 | 0.02 |
| 3 | 7.336 | 96294 | 0.62 |
| 4 | 8.800 | 30923 | 0.20 |
| 5 | 9.653 | 63346 | 0.41 |
| 6 | 9.981 | 14592 | 0.09 |
| 7 | 10.447 | 15349 | 0.10 |
| 8 | 11.440 | 95758 | 0.62 |
| 9 | 11.877 | 132292 | 0.86 |
| 10 | 12.207 | 217422 | 1.41 |
| 11 | 13.066 | 6246235 | 40.40 |
| 12 | 13.486 | 2272194 | 14.69 |
| 13 | 14.046 | 2436344 | 15.76 |
| 14 | 14.868 | 472685 | 3.06 |
| 15 | 15.527 | 370660 | 2.40 |
| 16 | 16.669 | 658939 | 4.26 |
| 17 | 17.230 | 168242 | 1.09 |
| 18 | 17.739 | 917577 | 5.93 |
| 19 | 18.501 | 735984 | 4.76 |
| 20 | 19.546 | 270981 | 1.75 |
| 21 | 20.324 | 141662 | 0.92 |
| 22 | 21.023 | 98337 | 0.64 |

**Table 4B Main peak areas and retention time in chromatography detection**

| # | Retention time | Peak area | % peak area |
|---|---|---|---|
| 1 | 7.295 | 2536 | 0.02 |
| 2 | 7.619 | 1584 | 0.01 |
| 3 | 7.643 | 1159 | 0.01 |
| 4 | 8.441 | 24235 | 0.20 |
| 5 | 8.945 | 1332 | 0.01 |
| 6 | 9.322 | 46100 | 0.37 |
| 7 | 9.671 | 18693 | 0.15 |
| 8 | 10.116 | 12874 | 0.10 |
| 9 | 10.464 | 6401 | 0.05 |
| 10 | 11.134 | 100618 | 0.82 |
| 11 | 11.542 | 107712 | 0.87 |
| 12 | 11.924 | 84129 | 0.68 |
| 13 | 12.079 | 72595 | 0.59 |
| 14 | 12.838 | 6335431 | 51.42 |
| 15 | 13.381 | 1267742 | 10.29 |
| 16 | 13.822 | 1891466 | 15.35 |
| 17 | 14.168 | 547291 | 4.44 |
| 18 | 14.719 | 330588 | 2.68 |
| 19 | 15.360 | 275838 | 2.24 |
| 20 | 15.856 | 33956 | 0.28 |
| 21 | 16.134 | 93890 | 0.76 |
| 22 | 16.482 | 398809 | 3.24 |
| 23 | 16.969 | 95196 | 0.77 |
| 24 | 17.577 | 397227 | 3.22 |
| 25 | 18.013 | 54857 | 0.45 |
| 26 | 18.401 | 119467 | 0.97 |

**Table 4C Main peak areas and retention time in chromatography detection**

| # | Retention time | Peak area | % peak area |
|---|---|---|---|
| 1 | 7.290 | 2542 | 0.02 |
| 2 | 7.614 | 1385 | 0.01 |
| 3 | 7.642 | 1119 | 0.01 |
| 4 | 8.405 | 26003 | 0.20 |
| 5 | 8.947 | 1558 | 0.01 |
| 6 | 9.338 | 49743 | 0.38 |
| 7 | 9.676 | 21486 | 0.16 |
| 8 | 10.137 | 11248 | 0.09 |
| 9 | 10.509 | 6231 | 0.05 |
| 10 | 11.161 | 80814 | 0.62 |
| 11 | 11.552 | 115175 | 0.88 |
| 12 | 11.919 | 201458 | 1.54 |
| 13 | 12.833 | 6554868 | 50.05 |
| 14 | 13.352 | 1500343 | 11.46 |
| 15 | 13.827 | 2051646 | 15.67 |
| 16 | 14.165 | 580765 | 4.43 |
| 17 | 14.695 | 409681 | 3.13 |
| 18 | 15.405 | 186479 | 1.42 |
| 19 | 15.716 | 133971 | 1.02 |
| 20 | 16.110 | 89273 | 0.68 |
| 21 | 16.466 | 367821 | 2.81 |
| 22 | 16.971 | 120239 | 0.92 |
| 23 | 17.569 | 353694 | 2.70 |
| 24 | 17.854 | 50995 | 0.39 |
| 25 | 17.973 | 54941 | 0.42 |
| 26 | 18.382 | 121957 | 0.93 |

**Table 5A Main peak areas and retention time in chromatography detection**

| # | Retention time | Peak area | % peak area |
|---|---|---|---|
| 1 | 7.153 | 43402 | 0.50 |
| 2 | 8.547 | 10401 | 0.12 |
| 3 | 9.409 | 28985 | 0.34 |
| 4 | 10.141 | 3161 | 0.04 |
| 5 | 11.207 | 31408 | 0.37 |
| 6 | 11.584 | 59171 | 0.69 |
| 7 | 11.962 | 47234 | 0.55 |
| 8 | 12.828 | 3525420 | 40.98 |
| 9 | 13.249 | 1256739 | 14.61 |
| 10 | 13.820 | 1511732 | 17.57 |
| 11 | 14.646 | 279064 | 3.24 |
| 12 | 15.240 | 205973 | 2.39 |
| 13 | 16.454 | 370498 | 4.31 |
| 14 | 17.510 | 552834 | 6.43 |
| 15 | 18.300 | 264290 | 3.07 |
| 16 | 18.893 | 119583 | 1.39 |
| 17 | 19.338 | 137776 | 1.60 |
| 18 | 20.046 | 88628 | 1.03 |
| 19 | 20.821 | 42519 | 0.49 |
| 20 | 21.517 | 23344 | 0.27 |

**Table 5B Main peak areas and retention time in chromatography detection**

| # | Retention time | Peak area | % peak area |
|---|---|---|---|
| 1 | 7.105 | 44323 | 0.44 |
| 2 | 8.487 | 12383 | 0.12 |
| 3 | 9.382 | 25154 | 0.25 |
| 4 | 9.738 | 4008 | 0.04 |
| 5 | 10.096 | 5892 | 0.06 |
| 6 | 10.462 | 3146 | 0.03 |
| 7 | 11.132 | 39277 | 0.39 |
| 8 | 11.532 | 51520 | 0.51 |
| 9 | 11.867 | 48987 | 0.49 |
| 10 | 12.749 | 3568760 | 35.41 |
| 11 | 13.174 | 1772217 | 17.58 |
| 12 | 13.732 | 1090494 | 10.82 |
| 13 | 14.101 | 417898 | 4.15 |
| 14 | 14.555 | 277829 | 2.76 |
| 15 | 15.211 | 175373 | 1.74 |
| 16 | 16.366 | 535098 | 5.31 |
| 17 | 17.420 | 472387 | 4.69 |
| 18 | 17.448 | 387633 | 3.85 |
| 19 | 18.199 | 539587 | 5.35 |
| 20 | 19.199 | 410304 | 4.07 |
| 21 | 20.267 | 87937 | 0.87 |
| 22 | 20.763 | 109568 | 1.09 |

**Table 5C Main peak areas and retention time in chromatography detection**

| # | Retention time | Peak area | % peak area |
|---|---|---|---|
| 1 | 7.197 | 2058 | 0.03 |
| 2 | 7.586 | 960 | 0.01 |
| 3 | 7.627 | 1039 | 0.01 |
| 4 | 8.390 | 17399 | 0.22 |
| 5 | 8.935 | 1431 | 0.02 |
| 6 | 9.296 | 30156 | 0.38 |
| 7 | 9.673 | 23883 | 0.30 |
| 8 | 10.159 | 6689 | 0.09 |
| 9 | 10.513 | 4025 | 0.05 |
| 10 | 10.864 | 2450 | 0.03 |
| 11 | 11.120 | 39285 | 0.50 |
| 12 | 11.560 | 64445 | 0.82 |
| 13 | 11.918 | 141948 | 1.81 |
| 14 | 12.827 | 3726812 | 47.46 |
| 15 | 13.260 | 1169681 | 14.90 |
| 16 | 13.830 | 1192710 | 15.19 |
| 17 | 14.172 | 363760 | 4.63 |
| 18 | 14.667 | 241046 | 3.07 |
| 19 | 15.381 | 102318 | 1.30 |
| 20 | 15.673 | 45485 | 0.58 |
| 21 | 15.927 | 4970 | 0.06 |
| 22 | 16.170 | 36141 | 0.46 |
| 23 | 16.509 | 215523 | 2.74 |
| 24 | 16.941 | 45912 | 0.58 |
| 25 | 17.322 | 32154 | 0.41 |
| 26 | 17.582 | 166907 | 2.13 |
| 27 | 17.837 | 67783 | 0.86 |
| 28 | 18.439 | 104829 | 1.34 |

### Comparative 3 Expression of proinsulin glargine containing 1 mutated fusion peptide sequence

The strategy in the Embodiment 1 was adjusted, the sequence for encoding insulin glargine was designed, and was expressed in a host cell, so that the proinsulin glargine contained a fusion peptide R sequence subjected to site-directed mutagenesis on 1 site on the basis of SEQ ID NO: 15. The amino acid sequence was designed as:
MATKAVSVLKGDGPVQGIINFEQKESNGPVKVWGSIHGLTEGLHGFHVHEFGDNTAGSTS AGPRFVNQHLCGSHLVEALYLVCGERGFFYTPKTRRGIVEQCCTSICSLYQLENYCG (SEQ ID NO: 16); and the nucleotide sequence for encoding the amino acid sequence was disclosed as SEQ ID NO: 19.

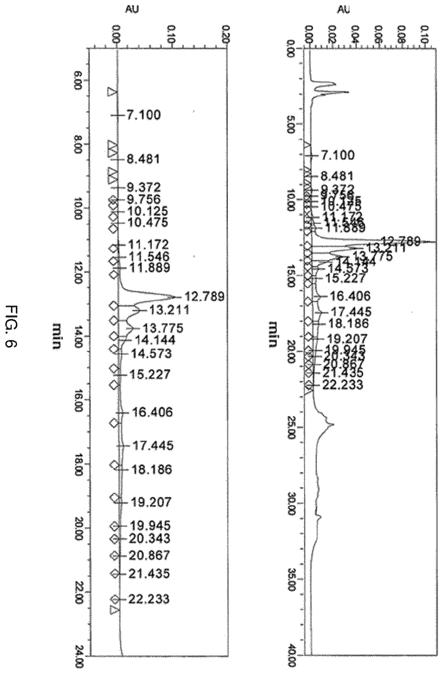

Renaturation was carried out according to the method in Embodiment 5. The yield of the proinsulin glargine renaturation precursor obtained by strain fermentation was detected by HPLC, and the result indicated that the yield of the proinsulin glargine renaturation precursor under the same conditions was 1.9 g/L. The renaturation liquid chromatography is respectively disclosed as FIG. 6, and the related data are disclosed as Table 6.

**Table 6 Main peak areas and retention time in chromatography detection**

| # | Retention time | Peak area | % peak area |
|---|---|---|---|
| 1 | 7.100 | 31530 | 0.58 |
| 2 | 8.481 | 6966 | 0.13 |
| 3 | 9.372 | 14681 | 0.27 |
| 4 | 9.756 | 2109 | 0.04 |
| 5 | 10.125 | 3795 | 0.07 |
| 6 | 10.475 | 3608 | 0.07 |
| 7 | 11.172 | 25035 | 0.46 |
| 8 | 11.546 | 42006 | 0.77 |
| 9 | 11.889 | 77271 | 1.42 |
| 10 | 12.789 | 1939000 | 35.64 |
| 11 | 13.211 | 814935 | 14.98 |
| 12 | 13.775 | 613260 | 11.27 |
| 13 | 14.144 | 231346 | 4.25 |
| 14 | 14.573 | 160525 | 2.95 |
| 15 | 15.227 | 96528 | 1.77 |
| 16 | 16.406 | 305068 | 5.61 |
| 17 | 17.445 | 459176 | 8.44 |
| 18 | 18.186 | 286812 | 5.27 |
| 19 | 19.207 | 180833 | 3.32 |
| 20 | 19.945 | 50905 | 0.94 |
| 21 | 20.343 | 48036 | 0.88 |
| 22 | 20.867 | 30012 | 0.55 |
| 23 | 21.435 | 15293 | 0.28 |
| 24 | 22.233 | 1649 | 0.03 |

### Comparative example 4 Expression of proinsulin glargine containing 2 mutated fusion peptide sequences

The strategy in the Embodiment 1 was adjusted, the sequence for encoding insulin glargine was designed, and was expressed in a host cell, so that the proinsulin glargine contained a fusion peptide R sequence subjected to site-directed mutagenesis on 2 sites on the basis of SEQ ID NO: 15. The amino acid sequence was respectively designed as:
MATKAVSVLKGDGPVQGIINFEQHESNGPVKVWGSIHGLTEGLHGFHVHEFGDNTAGSTS AGPRFVNQHLCGSHLVEALYLVCGERGFFYTPKTRRGIVEQCCTSICSLYQLENYCG (SEQ ID NO: 17); and the nucleotide sequence for encoding the amino acid sequence was disclosed as SEQ ID NO: 20.

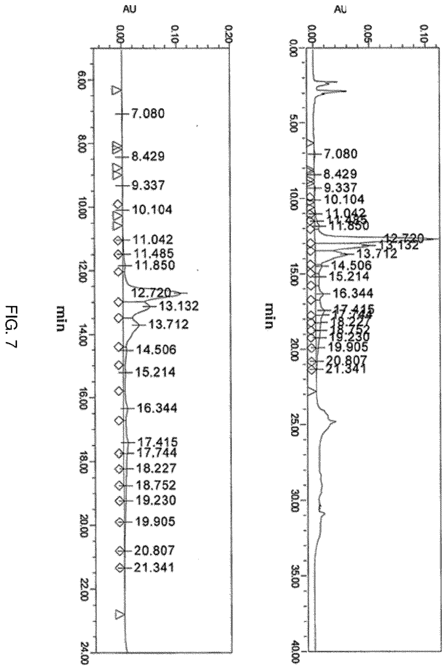

Renaturation was carried out according to the method in Embodiment 5. The yield of the proinsulin glargine renaturation precursor obtained by strain fermentation was detected by HPLC, and the result indicated that the yield of the proinsulin glargine renaturation precursor under the same conditions was 2.2 g/L. The renaturation liquid chromatography is respectively disclosed as FIG. 7, and the related data are disclosed as Table 7.

**Table 7 Main peak areas and retention time in chromatography detection**

| # | Retention time | Peak area | % peak area |
|---|---|---|---|
| 1 | 7.080 | 32442 | 0.54 |
| 2 | 8.429 | 7111 | 0.12 |
| 3 | 9.337 | 17594 | 0.29 |
| 4 | 10.104 | 1634 | 0.03 |
| 5 | 11.042 | 10294 | 0.17 |
| 6 | 11.485 | 36434 | 0.61 |
| 7 | 11.850 | 108086 | 1.80 |
| 8 | 12.720 | 2197231 | 36.59 |
| 9 | 13.132 | 1050997 | 17.50 |
| 10 | 13.712 | 931607 | 15.51 |
| 11 | 14.506 | 172841 | 2.88 |
| 12 | 15.214 | 174204 | 2.90 |
| 13 | 16.344 | 273268 | 4.55 |
| 14 | 17.415 | 359878 | 5.99 |
| 15 | 17.744 | 148993 | 2.48 |
| 16 | 18.227 | 137118 | 2.28 |
| 17 | 18.752 | 106493 | 1.77 |
| 18 | 19.230 | 109246 | 1.82 |
| 19 | 19.905 | 82369 | 1.37 |
| 20 | 20.807 | 25772 | 0.43 |
| 21 | 21.341 | 21575 | 0.36 |

### Comparative example 5 Expression of proinsulin glargine containing C peptide

The strategy in the Embodiment 1 was adjusted, and the sequence for encoding insulin glargine was designed, , and was expressed in a host cell, so that the proinsulin glargine contained "C peptide (EAR)"; the amino acid sequence was designed as:
MATHAVSVLKGDGPVQGIINFEQHESNGPVKVWGSIHGLTEGLHGFHVHEFGDNTAGSTS AGPRFVNQHLCGSHLVEALYLVCGERGFFYTPKTRREARGIVEQCCTSICSLYQLENYCG (SEQ ID NO: 18); and the nucleotide sequence for encoding the amino acid sequence was disclosed as SEQ ID NO: 21.

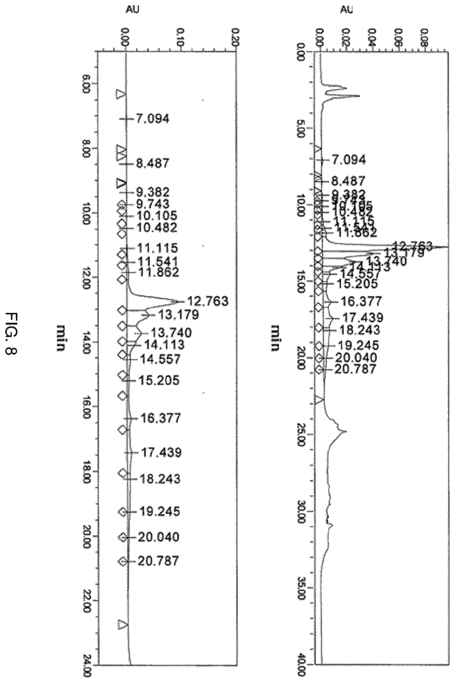

Renaturation was carried out according to the method in Embodiment 5. The yield of the proinsulin glargine renaturation precursor obtained by strain fermentation was detected by HPLC. The result is disclosed as Table 8. The renaturation liquid chromatography is disclosed as FIG. 8, and the related data are disclosed as Table 9.

**Table 8 Yields of proinsulin glargine renaturation precursor after different insulin glargine sequence expressions**

| Yield under use of WCB01 (g/L) | Yield of strain expressing SEQ ID NO: 19 (g/L) | Yield of strain expressing SEQ ID NO: 20 (g/L) | Yield of strain expressing SEQ ID NO: 21 (g/L) |
|---|---|---|---|
| 6.2 | 1.9 | 2.2 | 1.8 |

The result of Table 6 indicates that the fermentation yield obtained by using the fusion peptide sequence subjected to site-directed mutagenesis on 1 or 2 sites and the fusion peptide sequence adopting the "C peptide" (REA) strategy is far lower than the yield of WCB01.

**Table 9 Main peak areas and retention time in chromatography detection**

| # | Retention time | Peak area | % peak area |
|---|---|---|---|
| 1 | 7.094 | 30377 | 0.57 |
| 2 | 8.487 | 7761 | 0.15 |
| 3 | 9.382 | 14594 | 0.27 |
| 4 | 9.743 | 2603 | 0.05 |
| 5 | 10.105 | 4467 | 0.08 |
| 6 | 10.482 | 3140 | 0.06 |
| 7 | 11.115 | 25287 | 0.48 |
| 8 | 11.541 | 33463 | 0.63 |
| 9 | 11.862 | 75536 | 1.42 |
| 10 | 12.763 | 1878930 | 35.33 |
| 11 | 13.179 | 848037 | 15.95 |
| 12 | 13.740 | 611936 | 11.51 |
| 13 | 14.113 | 245888 | 4.62 |
| 14 | 14.557 | 172803 | 3.25 |
| 15 | 15.205 | 112415 | 2.11 |
| 16 | 16.377 | 259256 | 4.87 |
| 17 | 17.439 | 418883 | 7.88 |
| 18 | 18.243 | 305379 | 5.74 |
| 19 | 19.245 | 131360 | 2.47 |
| 20 | 20.040 | 73644 | 1.44 |
| 21 | 20.787 | 59947 | 1.13 |

### Comparative example 6: Renaturation, enzyme digestion transformation and purification

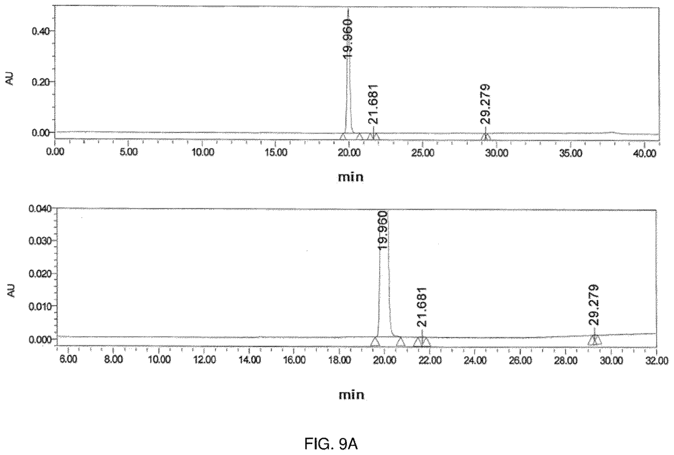

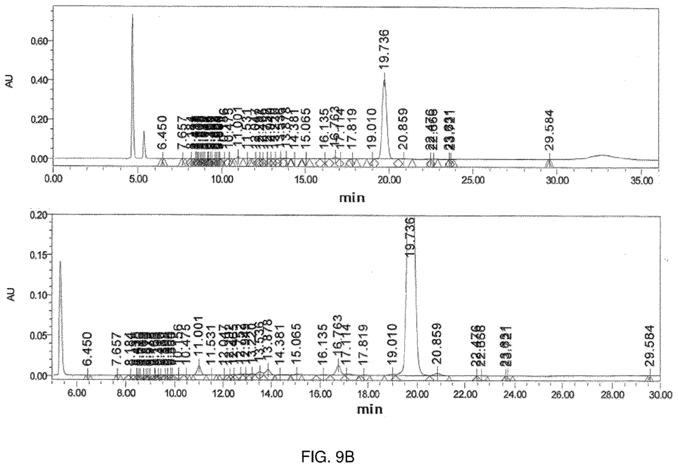

The inclusion body dissolving solutions prepared in the Comparative example 2 were respectively renatured according to the conditions in the Embodiment 5. Modification, enzyme digestion and purification were carried out on the renatured sample according to the method in Embodiment 6. The liquid chromatograms of the recombinant bacterium WCB01 and the recombinant bacterium WCB02 in the Comparative example 2 after sample purification are respectively disclosed as FIG. 9A and FIG. 9B, and related data are respectively disclosed as Tables 10A and 10B. The result indicates that the strain (WCB01) can gain the high main peak chromatographic purity of 99.93% and the maximum single impurity chromatographic purity of 0.05% by using the SOD fragment subjected to site-directed mutagenesis of 3 amino acids as the fusion peptide and utilizing the "0 C peptide" strategy. Meanwhile, the main peak and maximum single impurity chromatographic purities of the strain (WCB02) using the non-mutated SOD fragment as the fusion peptide are respectively 92.25% and 2.15%. By using the mutated SOD fragment and adopting the "0 C peptide" strategy, the main peak purity is enhanced a little, but the maximum single impurity content is obviously lowered by one order of magnitude, thereby avoiding the remaining of the C-peptide residues and reducing the quality loss and miscleavage impurities in the enzyme digestion transformation.

**Table 10A Main peak areas and retention time in chromatography detection**

| # | Retention time | Peak area | % peak area |
|---|---|---|---|
| 1 | 19.960 | 6005658 | 99.93 |
| 2 | 21.681 | 3011 | 0.05 |
| 3 | 29.279 | 1181 | 0.02 |

**Table 10B Main peak areas and retention time in chromatography detection**

| # | Retention time | Peak area | % peak area |
|---|---|---|---|
| 1 | 6.450 | 753 | 0.01 |
| 2 | 7.657 | 420 | 0.01 |
| 3 | 80184 | 609 | 0.01 |
| 4 | 8.438 | 538 | 0.01 |
| 5 | 8.510 | 268 | 0.00 |
| 6 | 8.576 | 256 | 0.00 |
| 7 | 8.709 | 792 | 0.01 |
| 8 | 8.816 | 316 | 0.00 |
| 9 | 8.879 | 360 | 0.00 |
| 10 | 8.972 | 987 | 0.01 |
| 11 | 9.162 | 449 | 0.01 |
| 12 | 9.186 | 268 | 0.00 |
| 13 | 9.304 | 365 | 0.00 |
| 14 | 9.410 | 1019 | 0.01 |
| 15 | 9.598 | 450 | 0.01 |
| 16 | 9.694 | 625 | 0.01 |
| 17 | 9.804 | 741 | 0.01 |
| 18 | 9.880 | 559 | 0.01 |
| 19 | 10.156 | 2983 | 0.04 |
| 20 | 10.475 | 739 | 0.01 |
| 21 | 11.001 | 119807 | 1.49 |
| 22 | 11.531 | 2151 | 0.03 |
| 23 | 12.047 | 1591 | 0.02 |
| 24 | 12.292 | 1474 | 0.02 |
| 25 | 12.465 | 2309 | 0.03 |
| 26 | 12.752 | 14885 | 0.18 |
| 27 | 12.949 | 15308 | 0.19 |
| 28 | 13.220 | 17592 | 0.22 |
| 29 | 13.536 | 52838 | 0.66 |
| 30 | 13.878 | 85586 | 1.06 |
| 31 | 14.381 | 8191 | 0.10 |
| 32 | 15.065 | 10546 | 0.13 |
| 33 | 16.135 | 8074 | 0.10 |
| 34 | 16.763 | 173201 | 2.15 |
| 35 | 17.114 | 21542 | 0.27 |
| 36 | 17.819 | 1731 | 0.02 |
| 37 | 19.010 | 20447 | 0.25 |
| 38 | 19.736 | 7428281 | 92.25 |
| 39 | 20.859 | 50936 | 0.63 |
| 40 | 22.476 | 53 | 0.00 |
| 41 | 22.666 | 1348 | 0.02 |
| 42 | 23.631 | 109 | 0.00 |
| 43 | 23.721 | 434 | 0.01 |
| 44 | 29.584 | 191 | 0.00 |

### Comparative 7: Enzyme digestion transformation using porcine trypsin

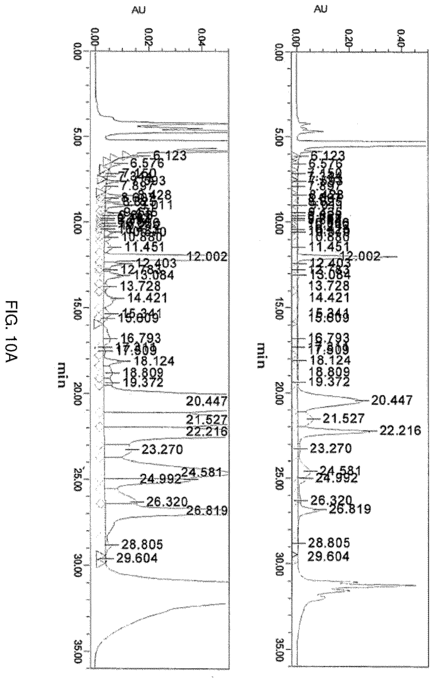

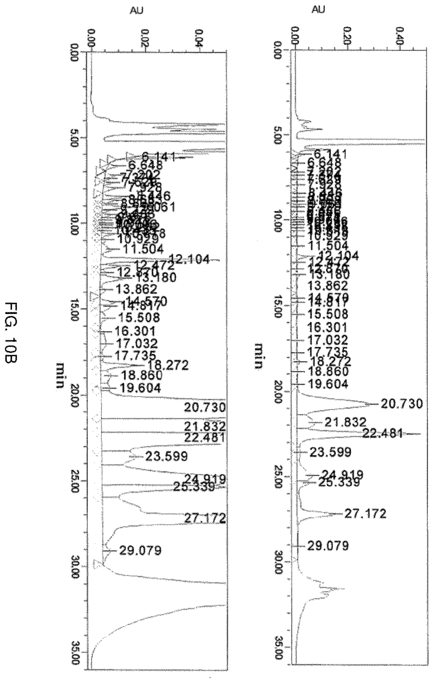

The WCB01 renatured solution prepared in the Embodiment 3 was subjected to enzyme digestion transformation through porcine trypsin according to the enzyme digestion method in Embodiment 4. The liquid chromatograms of the enzyme digestion transformation respectively utilizing porcine trypsin and bovine trypsin are respectively disclosed as FIG. 10A and FIG. 10B, and the related data are respectively disclosed as Table 11A and table 11B. The main peak area at the peak time of 20.447 min is 9,569,627 mAU*min as shown in FIG. 10A, and the main peak area at the peak time of 20.730 min is 11,142,487 mAU*min as shown in FIG. 10B. The result indicates that the enzyme digestion transformation rate of the bovine trypsin can be enhanced by 16% as compared with the porcine trypsin. Since the product concentration is in direct proportion to the peak area, the transformation rate is calculated according to the following formula (11,142,487-9,569,627)/9,569,627=16%.

**Table 11A Main peak areas and retention time in chromatography detection**

| # | Retention time | Peak area | % peak area |
|---|---|---|---|
| 1 | 6.123 | 25974 | 0.09 |
| 2 | 6.576 | 32496 | 0.12 |
| 3 | 7.150 | 18545 | 0.07 |
| 4 | 7.344 | 2774 | 0.01 |
| 5 | 7.593 | 36611 | 0.13 |
| 6 | 7.897 | 26753 | 0.10 |
| 7 | 8.428 | 65286 | 0.23 |
| 8 | 8.607 | 16521 | 0.06 |
| 9 | 8.825 | 15502 | 0.06 |
| 10 | 9.011 | 92535 | 0.33 |
| 11 | 9.435 | 27660 | 0.10 |
| 12 | 9.628 | 10516 | 0.04 |
| 13 | 9.787 | 5496 | 0.02 |
| 14 | 9.894 | 5612 | 0.02 |
| 15 | 10.066 | 9195 | 0.03 |
| 16 | 10.212 | 16404 | 0.06 |
| 17 | 10.423 | 6520 | 0.02 |
| 18 | 10.570 | 39115 | 0.14 |
| 19 | 10.880 | 29368 | 0.10 |
| 20 | 11.451 | 70108 | 0.25 |
| 21 | 12.002 | 3610530 | 12.83 |
| 22 | 12.403 | 98065 | 0.35 |
| 23 | 12.783 | 20620 | 0.07 |
| 24 | 130.84 | 125933 | 0.45 |
| 25 | 13.728 | 31204 | 0.11 |
| 26 | 14.421 | 134964 | 0.48 |
| 27 | 15.341 | 58382 | 0.21 |
| 28 | 15.609 | 18801 | 0.07 |
| 29 | 16.793 | 115174 | 0.41 |
| 30 | 17.311 | 2486 | 0.04 |
| 31 | 17.509 | 12968 | 0.05 |
| 32 | 18.124 | 111013 | 0.39 |
| 33 | 18.809 | 115239 | 0.41 |
| 34 | 19.372 | 70992 | 0.25 |
| 35 | 20.447 | 9569327 | 34.00 |
| 36 | 21.527 | 2327425 | 8.27 |
| 37 | 22.216 | 5483055 | 19.48 |
| 38 | 23.270 | 395684 | 1.41 |
| 39 | 24.581 | 2141960 | 7.61 |
| 40 | 24.992 | 611732 | 2.17 |
| 41 | 26.320 | 440323 | 1.56 |
| 42 | 26.819 | 2029210 | 7.21 |
| 43 | 28.805 | 61812 | 0.22 |
| 44 | 29.604 | 4203 | 0.01 |

**Table 11B Main peak areas and retention time in chromatography detection**

| # | Retention time | Peak area | % peak area |
|---|---|---|---|
| 1 | 6.141 | 96963 | 0.29 |
| 2 | 6.648 | 41805 | 0.13 |
| 3 | 7.202 | 38587 | 0.12 |
| 4 | 7.374 | 14189 | 0.04 |
| 5 | 7.629 | 21517 | 0.06 |
| 6 | 7.928 | 54454 | 0.16 |
| 7 | 8.446 | 73781 | 0.22 |
| 8 | 8.653 | 45482 | 0.14 |
| 9 | 8.868 | 21845 | 0.07 |
| 10 | 9.061 | 100605 | 0.30 |
| 11 | 9.229 | 18851 | 0.06 |
| 12 | 9.486 | 30588 | 0.09 |
| 13 | 9.675 | 14226 | 0.04 |
| 14 | 9.821 | 7601 | 0.02 |
| 15 | 9.973 | 11309 | 0.03 |
| 16 | 10.093 | 8934 | 0.03 |
| 17 | 10.252 | 18843 | 0.06 |
| 18 | 10.435 | 6463 | 0.02 |
| 19 | 10.618 | 37421 | 0.11 |
| 20 | 10.929 | 24440 | 0.07 |
| 21 | 11.504 | 79186 | 0.24 |
| 22 | 12.104 | 475734 | 1.42 |
| 23 | 12.472 | 78628 | 0.24 |
| 24 | 12.870 | 33249 | 0.10 |
| 25 | 13.180 | 160861 | 0.48 |
| 26 | 13.862 | 35587 | 0.11 |
| 27 | 14.570 | 76881 | 0.23 |
| 28 | 14.817 | 73991 | 0.22 |
| 29 | 15.508 | 75852 | 0.23 |
| 30 | 16.301 | 20875 | 0.06 |
| 31 | 17.032 | 106983 | 0.32 |
| 32 | 17.735 | 29030 | 0.09 |
| 33 | 18.272 | 198861 | 0.60 |
| 34 | 18.860 | 131756 | 0.39 |
| 35 | 19.604 | 78034 | 0.23 |
| 36 | 20.730 | 11142487 | 33.37 |
| 37 | 21.832 | 2758566 | 8.26 |
| 38 | 22.481 | 9109465 | 27.28 |
| 39 | 23.599 | 505312 | 1.51 |
| 40 | 24.919 | 2404115 | 7.20 |
| 41 | 25.339 | 1005576 | 3.01 |
| 42 | 27.172 | 4038121 | 12.09 |
| 43 | 29.079 | 87112 | 0.26 |

Although the present invention has been disclosed as above with preferred embodiments, it is not intended to limit the present invention. Anyone who is familiar with the technology can make various changes and modifications without departing from the scope of the present invention. Therefore, the scope of protection of the present invention should be subject to that defined in the claims.

## Claims

1. Proinsulin glargine, comprising an amino acid sequence having the following structure:
(B₁-B₃₂)-(A₁₋A₂₀)-A₂₁;
wherein,
B₁-B₃₂ is formed by adding two arginine Arg residues behind a C-terminal of a B₃₀ site of B₁-B₃₀ in a Chain B of natural human insulin;
A₁-A₂₀ is an insulin Chain A having 20 amino acids; and
A₂₁ is glycine;
wherein the structure of the amino acid sequence is:
R-R₁-(B₁-B₃₂)-(A₁-A₂₀)-A₂₁,
wherein,
R-R₁ is a fusion peptide sequence, and the amino acid sequence of R is MATX₁AVSVLKGDGPVQGIINFEQX₂ESNGPVKVWGSIX₃GLTEGLHGFHVHEFGDNTAGS TSAGP; X₁ is proline or histidine; X₂ is proline or histidine; X₃ is proline or histidine; and
R₁ is arginine or lysine.

2. The proinsulin glargine according to claim 1, wherein the amino acid sequence of R is disclosed as SEQ ID NO: 2 or SEQ ID NO: 3.

3. An DNA for encoding proinsulin glargine according to any one of claims 1 to 2.

4. An expression vector containing the DNA according to claim 3.

5. Non-plant cells comprising the proinsulin glargine according to any one of claims 1 to 2 or non-plant cells containing the expression vector according to claim 4.

6. A method for producing insulin glargine, comprising the following step: fermenting recombinant *Escherichia coli* expressing the proinsulin glargine according to any one of claims 1 to 2 at 35-37°C for at least 20 h to produce the insulin glargine.

7. The method according to claim 6, wherein the fermented insulin glargine is subjected to enzyme digestion, modification, renaturation and purification.

8. The method according to claim 7, wherein trypsin is used for the enzyme digestion; and citraconic anhydride is used for the modification.

9. Application of the method according to any one of claims 6 to 8 in preparation of insulin glargine or a drug containing the insulin glargine.

## Patentansprüche

1. Proinsulin glargin, umfassend eine Aminosäuresequenz mit der folgenden Struktur:
(B₁-B₃₂)- (A₁-A₂₀)-A₂₁;
wobei
B₁-B₃₂ durch Hinzufügen von zwei Arginin-Arg-Resten hinter einem C-Terminus einer B₃₀-Stelle von B₁-B₃₀ in einer B-Kette von natürlichem Humaninsulin gebildet wird;
A₁-A₂₀ eine A-Insulinkette mit 20 Aminosäuren ist; und
A₂₁ Glycin ist;
wobei die Struktur der Aminosäuresequenz wie folgt ist:
R-R₁-(B₁-B₃₂)- (A₁-A₂₀)-A₂₁,
wobei
R-R₁ eine Fusionspeptidsequenz ist und die Aminosäuresequenz von R MATX₁AVSVLKGDGPVQGIINFEQX₂ESNGPVKVWGSIX₃GLTEGLHGFHVHEFGDNT AGSTSAGP ist; X₁ Prolin oder Histidin ist; X₂ Prolin oder Histidin ist; X₃ Prolin oder Histidin ist; und R₁ Arginin oder Lysin ist.

2. Proinsulin glargin nach Anspruch 1, wobei die Aminosäuresequenz von R als SEQ ID NO: 2 oder SEQ ID NO: 3 angegeben ist.

3. DNA zum Codieren von Proinsulin glargin nach einem der Ansprüche 1 bis 2.

4. Expressionsvektor, der die DNA nach Anspruch 3 enthält.

5. Nicht pflanzliche Zellen, die das Proinsulin glargin nach einem der Ansprüche 1 bis 2 umfassen, oder nicht pflanzliche Zellen, die den Expressionsvektor nach Anspruch 4 enthalten.

6. Verfahren zum Herstellen von Insulin glargin, das den folgenden Schritt umfasst: Fermentieren von rekombinantem *Escherichia coli*, das das Proinsulin glargin nach einem der Ansprüche 1 bis 2 exprimiert, bei 35-37 °C für mindestens 20 Stunden, um das Insulin glargin herzustellen.

7. Verfahren nach Anspruch 6, wobei das fermentierte Insulin glargin einer enzymatischen Verdauung, Modifizierung, Renaturierung und Reinigung unterzogen wird.

8. Verfahren nach Anspruch 7, wobei Trypsin für die enzymatische Verdauung verwendet wird; und Citraconsäureanhydrid für die Modifizierung verwendet wird.

9. Anwendung des Verfahrens nach einem der Ansprüche 6 bis 8 bei der Herstellung von Insulin glargin oder eines Arzneimittels, das Insulin glargin enthält.

## Revendications

1. Proinsuline glargine, comprenant une séquence d'acides aminés ayant la structure suivante :
(B₁-B₃₂)-(A₁-A₂₀)-A₂₁ ;
dans laquelle,
B₁-B₃₂ est formé en ajoutant deux résidus arginine Arg derrière une extrémité C-terminale d'un site B₃₀ de B₁-B₃₀ dans une chaîne B d'insuline humaine naturelle ;
A₁-A₂₀ est une chaîne A d'insuline ayant 20 acides aminés ; et
A₂₁ est une glycine ;
dans laquelle la structure de la séquence d'acides aminés est :
R-R₁-(B₁-B₃₂)-(A₁-A₂₀)-A₂₁,
dans laquelle,
R-R₁ est une séquence peptidique de fusion, et la séquence d'acides aminés de R est MATX,AVSVLKGDGPVQGI MATX₁AVSVLKGDGPVQGIINFEQX₂ESNGPVKVWGSIX₃GLTEGLHGFHVHEFGDNT AGSTSAGP ; X₁ est une proline ou histidine ; X₂ est une proline ou histidine ; X₃ est une proline ou histidine ; et
R₁ est une arginine ou lysine.

2. Proinsuline glargine selon la revendication 1, dans laquelle la séquence d'acides aminés de R est décrite comme SEQ ID NO : 2 ou SEQ ID NO : 3.

3. ADN pour coder pour une proinsuline glargine selon l'une quelconque des revendications 1 à 2.

4. Vecteur d'expression contenant l'ADN selon la revendication 3.

5. Cellules non végétales comprenant la proinsuline glargine selon l'une quelconque des revendications 1 à 2 ou cellules non végétales contenant le vecteur d'expression selon la revendication 4.

6. Procédé de production d'insuline glargine, comprenant l'étape suivante : la fermentation *d'Escherichia coli* recombinant exprimant la proinsuline glargine selon l'une quelconque des revendications 1 à 2 entre 35 et 37 °C pendant au moins 20 h pour produire l'insuline glargine.

7. Procédé selon la revendication 6, dans lequel l'insuline glargine fermentée est soumise à une digestion enzymatique, une modification, une renaturation et une purification.

8. Procédé selon la revendication 7, dans lequel une trypsine est utilisée pour la digestion enzymatique ; et un anhydride citraconique est utilisé pour la modification.

9. Application du procédé selon l'une quelconque des revendications 6 à 8 pour la préparation d'insuline glargine ou d'un médicament contenant l'insuline glargine.
